# EUROPEAN PATENT SPECIFICATION

(11) **EP 4 545 042 B1**
(45) Date of publication and mention of the grant of the patent: **01.10.2025**
(21) Application number: 24215594.3
(22) Date of filing: 06.08.2021
(51) Int. Cl.: A61C 7/08, A61F 5/56, A63B 71/08, A61K 9/00, A61Q 11/00, B29C 48/00

(54) **PROCESS FOR FABRICATING A DENTAL APPLIANCE**
VERFAHREN ZUR HERSTELLUNG EINER DENTALVORRICHTUNG
PROCÉDÉ DE FABRICATION D'UN APPAREIL DENTAIRE

(30) Priority: 14.08.2020 EP 20191189
(43) Date of publication of application: 30.04.2025
(62) Divisional of application: 21763262.9
(73) Proprietor: Bussmedical AG, 6004 Luzern (CH)
(72) Inventor: Töpper, Tino, 79115 Freiburg (DE); Osmani, Bekim, 4056 Basel (CH); Dard, Elise, 4055 Basel (CH)
(74) Representative: Mertzlufft-Paufler, Cornelius

(56) References cited:
- WO-A1-2021/102260
- US-A1- 2002 083 955
- US-A1- 2005 100 853
- US-A1- 2008 293 007
- US-A1- 2013 259 920
- US-A1- 2018 344 579

## Description

The present disclosure relates to a method for initially loading or re-loading a dental appliance featuring a solid core of thermoplastic material with an agent, which is releasable from a functional coating of the dental appliance in aqueous environment. The disclosure also concerns a corresponding use of a reload-liquid.

Related to this particular disclosure is a dental appliance thermoformed from a thermoplastic functional foil comprising a core of thermoplastic material, in particular a core made from a thermoplastic polymer, and a thermoplastic coating at least partially covering the core. The dental appliance may be used as an orthodontic appliance or a protective appliance for protection of teeth and/or gingiva of the user of the appliance and said agent may be loaded or re-loaded into said coating of the appliance.

A dental appliance, as described before, can be worn by a user for protecting his teeth and/or gingiva, in particular from cigarette smoke and/or from resulting gingivitis and/or parodontitis; moreover, by wearing the appliance on the teeth, the formation of plaque can be reduced and thus the risk to develop caries.

In the past, intraoral orthodontic devices have been used in orthodontic therapy for realigning teeth. In this specific application case, the devices are typically designed as a dental splint, which is applied to teeth over several hours. The dental splint exerts forces on the individual teeth of the patient, which results in a realignment of the teeth in directions of desired teeth positions. For this purpose, typically a number of dental splints, which vary slightly from each other in shape, are worn by the patient consecutively to realign the teeth step-wise.

Further known applications of such intraoral orthodontic devices, in particular dental splints, are bleaching of teeth using bleaching pastes which are applied to the teeth by the device, or use of such devices for treating snoring overnight.

Intraoral appliances such as orthodontic devices are also used for avoiding clenching and grinding. Chronic teeth clenching and teeth grinding can cause overuse of the muscles controlling the lower jaw, leading to pain from those muscles. The load on the joint itself can also cause changes inside the joint, leading to pain and limited opening of the mouth.

In all of these applications, providing a high wearing comfort of the device is of central importance for a high acceptance of the treatment by the patient and hence overall success of the therapy. But also when a dental appliance is worn purely for protection of teeth, a high wearing comfort is an important quality.

US 2008/293007 A1 discusses methods for intra-oral drug delivery and proposes to attach bio-active substances to an oral appliance. In detail, it is suggested to adhere a layer with embedded agent to a portion of a surface of the oral appliance. The layer can thus later release the agent into the mouth of the patient, when the patient is wearing the appliance in his mouth.

In this context, the invention aims at further enhancing the functionality and biocompatibility of such dental appliances and their safety of use. In particular, the invention aims at equipping such appliances with desirable added functionality.

In accordance with the present invention, a method is provided according to claim 1, which solves the afore-mentioned problem. According to the invention, the functional coating of the dental appliance is a thermoplastic coating at least partially covering the core and this functional coating has been thermoformed together with the core to form the dental appliance. Moreover, the invention proposes to either initially load or re-load the agent into said coating by using a reload-liquid that contains the agent. This step can be simply achieved by immersing the dental appliance in the reload-liquid to load or re-load the agent into the coating.

As an example, a thermoplastic functional foil as described at the beginning featuring a thermoplastic core and a thermoplastic coating at least partially, but preferably fully, covering the core may be formed with a process comprising the following steps: providing a carrier liquid comprising an organic polymer, which may preferably be a thermoplastic polymer; and applying the carrier liquid as the coating onto the core.

Particularly suitable materials for the core are polyethylene terephthalate (PET), in particular PETG, polycarbonate (PC), and polyurethane (PU). These materials may also be used in recycled form (rPET, rPU, rPET, rPETG).

In other words, such a coating may be applied in the form of liquid coating onto a solid core, which may have the form of a core foil, in particular onto both sides of such a core foil, and to solidify the liquid coating afterwards on the core to form a solid thermoplastic coating covering the core.

Through such a process, a highly uniform and homogenous coating of the core can be achieved, and both the core and the coating can be thermoformed together in a later process step, as will be explained in greater detail below. Another great advantage of this liquid coating approach is that the coating can provide new functionalities to the foil and dental appliances formed from the foil. In particular, as will be explained in greater detail below, an agent may be mixed into the carrier liquid forming the coating prior to solidifying the coating on the core.

The coating may be formed, alternatively, using a hot melt extrusion process and providing the carrier liquid as a carrier melt. Such a carrier melt may also be applied onto a solid core to form a functional coating.

As will become clear below, the coating may form a thermoplastic cap layer of a dental appliance fabricated from the foil. When antimicrobial protection is to be provided, it can be beneficial if the cap layer fully covers the core of the device, and hence the thermoplastic coating may fully cover the core of the functional foil.

In the following, we first detail some possibilities of obtaining a foil, from which a dental appliance as introduced in the beginning may be thermoformed:
For example, one example suggests to add an agent to the carrier liquid prior to forming the coating. Preferably, the agent may be added to the carrier liquid in the form of an agent liquid, as many agents can be obtained already in liquid form. Such an agent may be in particular an agent that is releasable from the coating in aqueous environments. This has the advantage that such an agent can be released from the coating into an oral cavity of a user that is wearing a dental appliance formed from the functional foil.

Another highly preferred example suggests that the agent is derived from a bio-based material. In other words, the agent may be derived from a renewable biological resource, in particular a material of non-fossil origin produced by a living organism. The term "bio-based material" may be understood here in the sense that a bio-based material may comprise a non-fossil carbon content containing a detectable portion of the carbon isotope ¹⁴C. Preferably, the non-fossil carbon content may constitute at least 20%, preferably at least 35%, most preferably more than 50% of the bio-based material. In other words, about 80% to 65% but preferably much less of the bio-based material can be based on other organic material, in particular polymers, derived from fossil sources such as crude oil.

As will be explained later, such a bio-based material may also be used as the material for the coating and/or such a bio-based material may fully cover a core of a dental appliance fabricated from said functional foil, with the core not being made from a bio-based material. In other words, at least part of the coating may be based on one or more bio-based materials. Preferably, however, the coating may be fabricated exclusively from bio-based materials.

Most preferably, the agent may be derived from an essential oil. Such an oil may be extracted directly from non-fossil plants. Such features ensure that the bio-based agent is not harmful to the body of the user of the dental appliance. Moreover, with such an approach, a myriad of different functionalities such as adding flavor or antimicrobial protection may be achieved by relying on natural products that do not lead to harmful contaminations inside the oral cavity.

According to another preferred example, the agent may comprise at least one of the following substances: an essential oil, an extract of an essential oil, or a derivative from the chemical family of phenols, monoterpenols, aldehydes, ketones, oxide terpenes, ethers, monoterpenes, lactone, phthalides, terpenic aldehydes, sestequiterpen alcohols, sestequiterpen, terpene esters, coumarins, in particular a substance such as cinnamaldehyde, lime, thymol, eugenol, linalool, carvacrol, nutmeg, pimenta berry, rosemary, petitgrain, coffee, anise, spearmint.

Further extracts/derivatives obtained from bio-based materials, in particular through extraction from a natural essential oil, that may be used as an agent to be embedded in the coating, are + α, β-pinene, myristicin, methyl eugenol, menthol, terpinene, p-cymene, linalool, myrcene, pinene, β-ocimene, geranial, sabinene, neral, citronellol, linolenic acid, oleic, stearic, himachalene, bisabolene, trans-cinnamaldehyde, methyl salicytate, eucalyptol, trans-anethole, (+)-limonene, (-)-limonene or L-carvone.

A particular advantage of using lime and cinnamaldehyde as agents is that they are not only highly effective antimicrobial agents but also provide a softening and plasticizing effect to the thermoplastic coating.

To further enhance the safety and compliance of dental appliances formed from the functional foil, it is highly preferable, if the organic polymer contained in the carrier liquid is a bio-based material or at least derived from a bio-based material. In other words, the organic polymer (forming the coating) may be derived from a renewable biological resource, in particular a material of non-fossil origin produced by a living organism.

A particular suitable choice for the organic polymer (forming the coating) is a cellulose-based material, in particular a cellulose-based composite. This is because cellulose is a bio-based material that can render the surface of the dental appliance soft, after swelling in water; in addition, cellulose offers favorable chemcial interactions with the mentioned agents to be loaded or reloaded into the CAP layer comprising cellulose based composites.

Suitable materials for fabricating the core of the foil are cellulose acetate, cellulose acetate butyrate, PLA, chitosan or a polyester, a co-polyester, polycarbonate, polyurethane, polypropylene, polyethylene, polypropylene and polyethylene copolymer, acrylic, cyclic block copolymer, polyether-etherketone, polyamide, polyethylene terephthalate, polybutylene terephthalate, polyetherimide, polyethersulfone, polytrimethylene terephthalate or a combination thereof (e.g., a blend of at least two of the listed hard polymeric materials). In some embodiments, the core of the device can include polymeric materials, such as polycarbonate, a co-polyester, a polyester, and polyurethane. Moreover, the core can also be composed of multiple layers, e.g., two or more polymer layers.

Preferably, however, the core should mainly consist of a thermoplastic polymer (to render the foil thermoformable).

Another highly preferred choice is to use cellulose acetate butyrate (CAB) as the organic polymer (i.e. for the coating). In this case, it is highly preferable for good mechanical properties of the thermoplastic functional foil (and dental appliances formed from the foil), if a butyryl content of the CAB is between 15 to 60% by weight.

By providing such features, it can be achieved that the agent embedded into the coating is releasable from the coating, in particular in aqueous environments, for example in the oral cavity. Moreover, it can be achieved by adequate choice of the agent, for example by using cinnamaldehyde as the agent, that the coating provides antimicrobial protection (through the release of an antimicrobial agent). Hence, the agent may be an antimicrobial agent.

To improve the malleability and adaptiveness of the foil during thermoforming, it is of advantage if a glass transition temperature T_{g,coating} of the coating, and/or in particular a glass transition temperature of the organic polymer T_{g,polymer}, is/are in the range of 80-165°C. This is particularly true when using CAB as the organic polymer.

A suitable corresponding melting range of the coating, and in particular said organic polymer, may be from 120-200°C.

Another important factor for achieving satisfying results when thermoforming the functional foil is the number average molecular weight of the organic polymer used for the coating. For optimum results, the number average molecular weight may be more than 12.000 g/mol, preferably more than 20.000 g/mol, and most preferably more than 30.000 g/mol. Such numbers are particularly suited if CAB is chosen as the organic polymer.

Another highly advantageous example of the process introduced at the beginning proposes that the carrier liquid is a carrier solution, i.e. the carrier liquid may be a solution of a solvent and the organic polymer. In this case, it if preferable if the carrier solution is obtained by dissolving the organic polymer in a solvent. This has the advantage that the coating may be solidified on the core by evaporating the solvent from the coating. This approach is of advantage because the solvent can be used to modify the surface of the core, as will be explained later. As already said, the core may actually be a core foil and hence, the carrier solution may be applied to both sides of the core foil uniformly.

For achieving a uniform coating thickness, the coating can be formed by a physical coating process such as spin coating or blade-based coating, or by spray coating or by roll-to-roll coating. Of course, there is a myriad of different processes are available for performing roll-to-roll coating **(e.g.** air knife coating, gap coating, immersion dip coating, roller coating, etc.) or spray coating (spray painting, thermal spraying, plasma spraying etc.), as examples.

As already mentioned, the solvent of the carrier solution can be used to modify the surface of the core. In fact, the solvent can soften the core's surface by reducing the interaction of molecular chains of the material of the core. As a result, after treating the core with the carrier solution, the core and the coating (or to be more precise, the organic polymer of the coating) can interlock on a nanometer scale, resulting in improved adhesion of the coating on the core. After application of the coating in liquid form onto the core and interaction of the solvent with the core, the solvent may be simply evaporated thus forming a final solid coating.

Such as softening of the core can be achieved, for example, when using acetone as the solvent and PETG as the core material, but there exist other combinations of core materials and suitable solvents, which achieve the same softening effect, as is readily apparent in the literature. The invention suggests to use this effect for enhancing the adhesion of the coating on the core of a functional foil intended for fabricating a dental appliance.

Another approach for improving the mechanical and/or chemical interaction of the coating and the core is to safeguard that a glass transition temperature T_{g,core} of the core and a glass transition temperature T_{g,coating} of the coating differ by less than 80°C, preferably by less than 60°C. Such a material choice will result in enough mobility of the molecular chains of both core and coating such that efficient thermal interlocking can be achieved through thermal fusion (sometimes referred to as "thermal welding") between core and coating during the final thermoforming of the functional foil.

Moreover, in such a case, it can be of advantage, if the glass transition temperature of the coating T_{g,coating} is higher than the glass transition temperature of the core T_{g,core}. Such a material choice will result in a sufficient mechanical stability during thermoforming of the device, as the organic polymer can provide some stability to the foil. For example, PETG, which is a useful material for the core, can have a glass transition temperature of 80°C, and a melting temperature as high as 210°C. Such a core can be combined with a coating based on cellulose acetate butyrate (CAB), and such a CAB-coating may feature a glass transition temperature T_{g,coating} of 120°C or above, but at the same time a relatively low melting temperature T_{m,coating} of 160°C.

Additionally, either the coating should reach its melting temperature T_{m,coating} before the core reaches its melting temperature T_{m,core} or the core should reach its melting temperature before the coating reaches its own. This may be achieved - in particular when considering thermoforming - by safeguarding that a melting temperature of the core T_{m,core} and a melting temperature of the coating T_{m,coating} differ by at least 20°C, preferable at least 40°C, most preferably at least 60°C. As a result, a compound material may be produced at the interface between core and coating during thermoforming of the functional foil, because when for example the coating reaches its melting temperature, the core may still be in the glass transition phase/temperature range (or vice versa). This compound material will thus comprise chains from the organic polymer of the thermoplastic coating and parts of the material used for the thermoplastic core.

Preferable for easy fabrication, however, will be a design, in which the melting temperature of the core T_{m,core} is at least 20°C, preferable at least 40°C, most preferably at least 60°C, higher than the melting temperature of the coating T_{m,coating}. In such a design, the coating will become molten while the core material is still solid or still in the glass transition stage. This is highly beneficial for obtaining a robust process control, when thermoforming the foil.

The solvent used for the carrier solution may comprise at least one of acetone, methyl acetate, ethyl acetate, methylethyl ketone, isopropyl acetate, butyl acetate, ethyl lactate, cyclohexane, diacetone alcohol, butyl lactate or suitable mixtures of these solvents.

Another variant is defined in that the agent itself may be a liquid. In this case, the agent in liquid form (agent liquid) may be simply mixed with the carrier liquid.

Alternatively, the agent may be added to the carrier liquid by dissolving or emulsifying the agent first in an aqueous or oil-based solution and mixing this solution (agent liquid) with the carrier liquid. In such a case, it is preferable for a homogenous distribution of the agent within the final coating, if the solution containing the agent is mixed with the carrier liquid, for example by a stirrer, to form a homogenous carrier solution, prior to forming the coating.

Concerning the ratio between the agent and the organic polymer in the final coating, this ratio may be typically between 0.01/99.99 and 30/70 by weight, for example 2-3g of agent and 7g of organic polymer. For most applications, it will be preferable if the ratio between the agent and the organic polymer is between 0.1/99.9 and 20/80 by weight.

A particular suitable process can be obtained, if the core of the functional foil is made from Polyethylenterephthalat (PET). In particular, the core may be made at least partly from recycled PET. In other words, the thermoplastic material of the core may comprise recycled PET.

For improving the mechanical properties of the functional foil, in particular for reducing the tendency for crack formation, glykol modified Polyethylenterephthalat(PETG) may be chosen as the material for the core. In these cases, a highly suitable solvent for achieving the softening effect described above is acetone.

The process may be further modified in that a natural softening agent, preferably triacetin or polycaprolactone-triol (PCL-T), is added as a plasticizer to the carrier liquid prior to forming the coating. This may be done, preferably, at a volume ratio of less than 40%, preferably of less than 20%, most preferably of less than 10 %.

Finally, the process explained so far may be continued by forming a dental appliance from the functional foil by thermoforming the foil. Importantly, this thermoforming may be done after application of the coating onto the core, as both the core and the coating may be thermoplastic. As a result, after thermoforming of the functional foil, the organic polymer may form a conformal functional coating, preferably fully covering the core of the foil, which then constitutes the core of the dental appliance. Moreover, an agent previously embedded in the coating (as described above) may then provide antimicrobial protection or regenerative functionality for teeth and gingiva or a flavor or simply a nice color to the dental appliance.

An important aspect with regard to the final thermoforming is the thermal stability of the agent. In order to safeguard the proper functionality of the final device, a decomposition temperature of the agent T_{d,agent} should be at least 10°C above the melting temperature T_{m,coating} of the coating (the decomposition temperature T_{d,agent} may thus lie up to 60°C above the glass transition temperature T_{g,coating} of the coating) .

Following the concept outlined above, the afore-mentioned problem underlying the invention is thus effectively solved by a dental appliance when it is initially loaded or re-loaded with an agent releasable from the functional coating of the appliance as detailed in claim 1. This dental appliance may be an orthodontic appliance or a protective appliance for protection of teeth and/or gingiva, and the appliance is thermoformed, for example from a functional foil fabricated with a process as described herein.

In such a dental appliance, the agent described above with respect to the functional foil or an agent embedded into the dental appliance (preferably into a cap layer of the appliance) may be releasable from the dental appliance during intra-oral use. In addition, the invention suggests, that such an agent can be reloaded into a cap layer of the dental appliance using a reload-liquid (to be described in more detail further below).

According to a particular approach intended for using the dental appliance purely for protection of teeth and gingiva, the dental appliance may consist entirely of the functional foil, which may have a low thickness of less than 400 µm, preferably of less than 300 µm, most preferably of less than 200 µm.

With respect to the properties of the final device, it may be described as featuring a core of thermoplastic material, a thermoplastic coating at least partially covering the core, and an agent embedded in the coating and releasable from the coating in aqueous environment. The core and/or coating and/or agent may be chosen and/or designed as has been described in detail above with respect to the fabrication process.

Most preferably, the agent should be embedded in the coating with a surface density of at least 0.05 mg of the agent per cm² surface area of the coating, because this guarantees a release rate sufficient for achieving the desired functionality of the appliance when worn in the mouth. Such a surface density may be easily reached by using a reload-liquid as described in detail further below.

When achieving such a surface density, a release rate of the agent from the dental appliance of at least 1 mg/m² surface area of the coating per hour over a period of 24 hours can be maintained, when the appliance is worn in the mouth; this is particularly true when using a cellulose-based coating and an agent such as Limonene, cinnamaldehyde, methyl Salicylate or trans-anethole. Such a release rate has been found to be adequate in particular when the agent is chosen to provide antimicrobial protection.

Due to its increased functionality, the dental appliance can be used for protecting teeth and/or gingiva of a user wearing the dental appliance on his/her teeth. This protection can be in particular from cigarette smoke and/or gingivitis and/or parodontitis and the dental appliance may be fabricated as previously described. The wearing of the protective dental appliance on the teeth can also provide protection from plaque formation and thus reduce the risk to develop caries. This is particularly true when using lime and cinnamaldehyde as an agent embedded in a coating of the dental appliance. The dental appliance may also be worn for cosmetic reasons, for example if used in combination with a bleaching agent, or as a night guard, for providing protection against plaque, bacteria and gingivitis and/or for providing good taste and smell.

During use, the dental appliance may cover both teeth and parts of the gingiva adjoining to the teeth, and said functional coating can offer antimicrobial protection. This protection is provided, as has been explained above, through the releasable antimicrobial agent and this agent may be embedded in the functional coating as described previously. The coating may be formed from a bio-based material and the agent may also be derived from a bio-based material. This further enhances the protection and safety when wearing the appliance.

It is preferable if the dental appliance is formed with cavities matching the natural positions of the teeth of a user such that the appliance does not exert forces onto the teeth. This greatly improves the wearing comfort, which is important, if the user wants to profit from long-time protection by wearing the dental appliance over hours. For the same reason, it is regarded highly preferable, if the thermoplastic functional foil shows a maximum thickness of less than 700 µm, preferably of less than 500 µm, most preferably of less than 300 µm (after the final thermoforming process). This is because, in particular due to the liquid coating approach, the thickness of the functional coating may be less than 100 µm or even less than 50 µm, while the coating may be still providing sufficient antimicrobial protection, due to a high concentration of the agent inside the coating. In particular, such a dental appliance can be comfortably worn by a user while the user is smoking or sleeping.

In the use case of dental teeth alignment the embedded agent may also serve as a plasticizer, which reduces the brittleness of the thermoformed dental appliance and enables a long-term stable flexibility during a typical wearing period of two weeks (e.g. for each aligner in a series of aligners).

For providing greater flexibility in the choice of the agent or for replenishing the agent in the coating, after the dental appliance has been used multiple times, a use of a re-load liquid is proposed according to claim 2: This reload-liquid can load the agent into the dental appliance, in particular into the coating/cap layer described previously.

As described in claim 1, such loading may be done initially in case no agent has been embedded in the appliance during its fabrication; or the reload-liquid can re-load an agent initially present in the coating, but consumed through release from the appliance into the mouth, again into the coating. Hence, the reload-liquid can be used both for loading or re-loading a dental appliance (which may be designed and/or fabricated as described previously) with a releasable agent. .

For achieving sufficient loading, it is preferable if the reload-liquid contains a concentration of the agent of at least 0.2 g/l. In this case, a surface density of the agent embedded in the dental appliance of 0.05 mg/cm² can be achieved. This may result in a release rate from the dental appliance, in particular from said coating / said cap layer, of at least 1 mg/m² per hour, when the dental appliance is worn in the mouth during a typical wearing period of at least 24 hours.

In addition, it is also possible to use such a liquid for loading a releasable agent into a thermoplastic functional foil, in particular prior to thermoforming.

The reload-liquid may be in the form of an aqueous solution or an oil-based liquid. It can also contain solvents such as ethanol. Such a solvent can reduce the surface tension and help in wetting the appliance with the reload liquid.

A particular convenient way is to obtain the reload-liquid from a liquid precursor comprising the agent (and preferably a surfactant to be described below), which may then be diluted to obtain the reload liquid.

An alternative approach is to obtain the reload-liquid by dissolving a solid tablet containing the agent in a liquid, preferably in water. Hence, it is proposed to use such a solid tablet for forming a reload-liquid and to use the load-liquid as described before. The tablet can therefore be soluble in water and the liquid used may be water.

The reload-liquid and/or the tablet just described may comprise a surfactant for homogenously distributing the agent within the reload-liquid. This is important for achieving a high load concentration of the agent per surface area of the coating.

One particularly suitable composition of such a tablet is a combination of an acidic material (e.g. citric acid), a base (e.g. sodium bicarbonate and citric acid), a fatty acid ester (e.g. Polyglyceryl-4 Laurate and/or Polyglyceryl-6 Caprate), a calcium derivative (e.g. calcium carbonate) and a sweetener (e.g. xylitol). To this composition, the agent or a combination of multiples agents, preferably and a surfactant (which may comprise a solubilizer and an emulsifier), may be added.

The surfactant may be derived from a renewable biological resource, in particular a material of non-fossil origin produced by a living organism. Preferred surfactants for this application are fatty acid esters, benzoic alcohols or glycerol-based surfactants. Further examples of surfactants particularly suitable for the application described herein are Polyglyceryl-4 Laurate or Polyglyceryl-6 Caprate. All of these substances can also be used in combination and have been found to be particularly suitable to be used with the agent cinnamaldehyde.

The concentration of surfactant and agent in the reload-liquid define the speed of loading the agent into the dental appliance. However, the ratio between surfactant and agent needs to be carefully balanced: On the one hand, the smaller the ratio of surfactant to agent, the faster the agent can diffuse from the reload-liquid into a cap layer of the dental appliance. In other words, the concentration of the surfactant should be as low as possible, since for excessive concentrations, the surfactant will capture the agent in the reload-liquid. On the other hand, the higher the concentration of surfactant and agent with respect to water in the reload liquid, the faster will be the (re-)loading.

A preferred ratio of the concentration cₛ of the surfactant and a concentration cₐ of the agent is cₛ/cₐ < 100, most preferably cₛ/cₐ < 10. Such ratios are particularly suited for the specific case of using Polyglyceryl-4 Laurate as an emulsifier and Polyglyceryl-6 Caprate as solubilizer (the surfactant thus comprises both of these components in this case). If the ratio cₛ/cₐ exceeds such values, there is a high likelihood that the agent will be captured (due to polar/non-polar interaction between single molecules) by the surfactant and not be embedded into the dental appliance.

Such parameters ensure, that a high absorption speed can be achieved, such that a significant amount of the agent (sufficient for producing a significant effect during wearing of the appliance) can be embedded into the coating of the dental appliance in less than an hour, or even less than 15 min (thus rendering the process of re-loading comfortable for the user). As a specific example, the amount of surfactant mₛ in the final reload-solution mₛₒₗ may be mₛ/mₛₒₗ > 0.1g/l.

Preferred examples of the present invention will now be described in more detail. With reference to the accompanying drawings, where features with corresponding technical function are referenced with same numerals even when these features differ in shape or design:
- Fig. 1: is a schematic overview of a fabrication process,
- Fig. 2: illustrates the definition of non-fossil carbon content and bio-based content used herein to describe bio-based materials,
- Fig. 3: schematically illustrates a method for loading an agent into a dental appliance using a reload-liquid according to the invention,
- Fig. 4: shows measurement data of concentrations of a particular agent loaded into different thermoplastic materials, and
- Fig. 5: depicts further experimental data of calorimetric measurements.

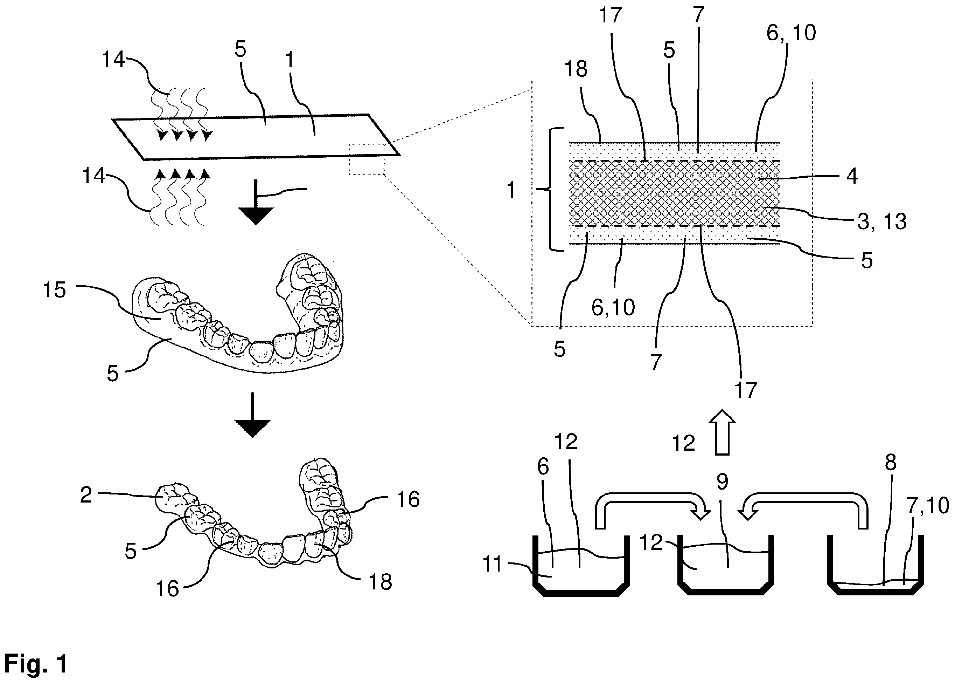

Figure 1 provides an overview of a suitable fabrication process for a foil, from which a dental appliance as discussed herein can be thermoformed: An organic polymer 6, namely cellulose acetate butyrate (CAB), is dissolved in a solvent 12, namely acetone, to form a carrier solution 11. The carrier solution 11 is next mixed with an agent liquid 8 containing cinnamaldehyde, which is a bio-based antimicrobial agent 7, to form a carrier liquid 9, in which the agent 7 and the CAB are homogenously mixed. This carrier liquid 9 is then applied by dip coating onto a core foil 13 of a thermoplastic material 4. After evaporation of the solvent 12, a solid thermoplastic coating 5 is obtained on both sides of the core-foil 13, which thus completely covers the core 3 of the resulting functional foil 1 (c.f. the cross-section visible in Figure 1).

The foil 1, in fact, constitutes a multi-layer structure with a core 3 made from glykol modified Polyethylenterephthalat (PETG) with a thickness of 200 µm and top and bottom functional coatings 5 which each have a thickness (after solidification) of less than 20 µm, thus resulting in a total thickness of the thermoplastic functional foil 1 of less than 250 µm.

As a next step, using a 3D-model of an oral cavity of a patient, a pre-form 15 is formed from the functional foil 1 by thermoforming, applying heat 14 from both sides to the foil 1 and thus distorting the core 3 and the coatings 5 together in one step. In other words, the coating 5 is already firmly linked to the core 3 prior to thermoforming. The underlying reason is that the acetone 12 modifies the surface of the PETG core 3 leading to a softening of the PETG surface such that the CAB molecules 6 can interlock on a nanometer scale at the interface 17 (cf. Figure 1) with the PETG of the core 3. As a result, there is already a high adhesion of the coating 5 on the core 3 prior to thermoforming.

As the glass transition temperatures T_{g,core} of the PETG-core 3 and T_{g,coating} of the coating differ by less than 60°C, during thermoforming the adhesion of the coating 5 on the core foil 13 is further improved, as a thermal interlocking is achieved through thermal fusion of the CAB-coating 5 with the PETG.

Finally, the pre-form 15 is cut such that the resulting dental appliance 2 visible on the bottom left of Figure 1 covers both teeth and adjoining parts of the gingiva. As visible, the dental appliance 2 further features multiple cavities 16 designed for taking up single teeth and which are matching the natural positions of the teeth of the user. Thus, the user experiences no forces on his teeth when wearing the dental appliance 2 on his teeth. As the appliance 2 is very thin, highly conformable and offers a soft and highly deformable functional coating 5, which, due to the CAB used, even softens when getting into contact with saliva and offers antimicrobial protection due to the embedded cinnamaldehyde, the appliance 2 is ideally suited to be worn overnight or while smoking, even for hours. In such non-therapeutic use-cases, the user can benefit from the protection provided by the appliance 2 against cigarette smoke, as the appliance 2 is impermeable to smoke, but also from gingivitis and parodontitis, due to the antimicrobial effect that is produced when the cinnamaldehyde 7 is slowly released from the coating 5 into the oral cavity of the user.

The CAB used for the coating 5 encapsulating the core 3 as illustrated on the right of Figure 1 is in fact a bio-based material 10, as it is produced by blending organic materials derived from fossil sources with organic material, in particular cellulose, derived from non-fossil natural sources such as plants. Therefore, the complete outer surface 18 of the appliance 2 is formed from bio-based materials 10. Any mechanical abrasion from this surface 18 will thus not be harmful for the user wearing the appliance 2 in his mouth. Likewise, the agent 7 embedded in the coating 5 can be derived from natural sources such as essential oils extracted directly from non-fossil plants. This allows use of agents such as lime, thymol, eugenol, linalool, carvacrol, nutmeg, pimenta berry, rosemary, petitgrain, coffee, anise, to name a few. Hence the appliance 2 can deliver both flavor and antimicrobial protection and still be highly biocompatible and non-harmful to wear.

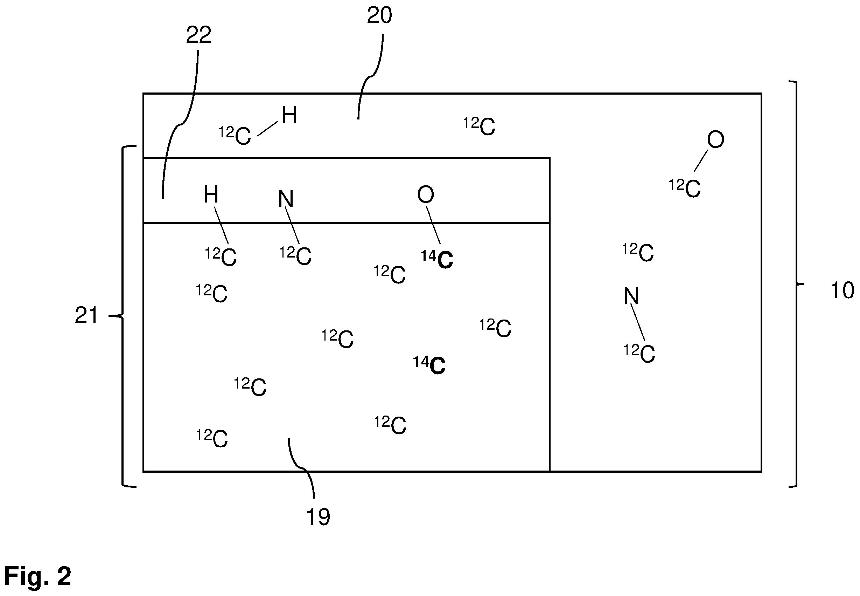

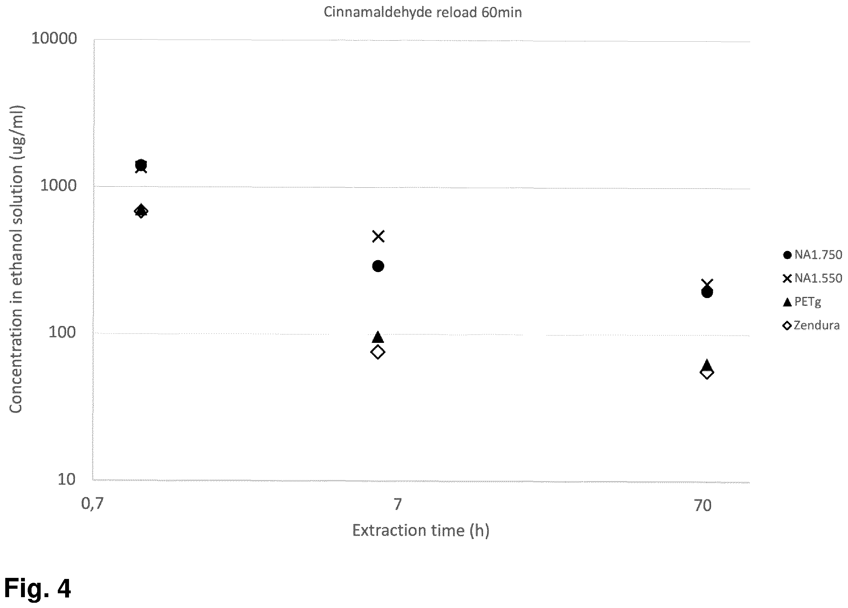

As Figure 2 illustrates schematically, "bio-based material" as understood herein may mean that the organic polymer 6 as well as the agent 7 used for the coating 5 may contain a significant portion of non-fossil carbon content 19, for example at least 40%. This non-fossil carbon content 19 is characterized in that it contains the carbon isotope ¹⁴C in a detectable fraction (e.g. more than 10 ppq or even more than 100 ppq). There may also be a fossil carbon content 20 (cf. Figure 4), which is based on fossil sources and containing the isotope ¹²C but no detectable fraction of ¹⁴C any more, as the ¹⁴C has been diminished by radioactive decay over thousands of years. As illustrated in Figure 4, there can be defined a further quantity namely the so-called bio-based content 21. This fraction of the material 5 comprises the non-fossil carbon content 19 as well as all hydrogen H-, oxygen O-, and nitrogen N-atoms 22 bound to the non-fossil carbon content 19.

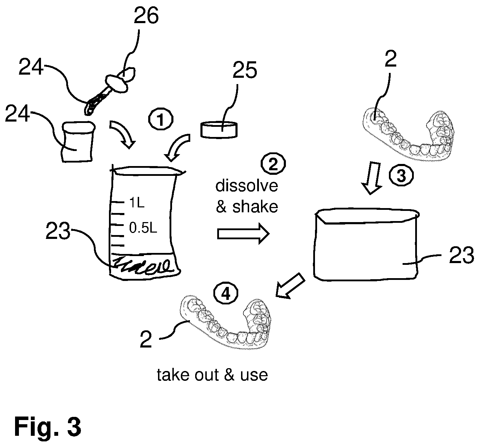

Figure 3 illustrates the formation of a reload-liquid and a use of such a reload-liquid as proposed by claims 1 and 2: In a first step, a highly concentrated liquid precursor 24 containing the agent 7 and a surfactant are first dispensed in water using a low-cost pipette 26; alternatively, a tab 25 containing the agent 7 and a surfactant may be dissolved in the water. By both ways, a reload-liquid 23 may be formed. In a second step, the reload-liquid 23 is shaken to allow thorough mixing of the liquid precursor 24 with the water and/or full dissolving of the tab 24 in the water.

In a third step, as proposed by claim 1, the dental appliance 2 is immersed and soaked in the formed reload-liquid 23 for a duration of 15-30 minutes. Finally, in a fourth step, the dental appliance 2 is taken out of the reload-liquid 23 and is now loaded with the agent 7 and ready for use in the mouth, where the agent 7 can be released from the dental appliance 2, for example for producing a pleasant taste and/or an antimicrobial effect.

Figure 4 displays experimental data obtained by first loading four different thermoplastic materials with an agent 7 (cinnamaldehyde) by immersing the respective thermoplastic material in a reload-liquid 23 (comprising 12 g of a surfactant and 0.56 g of the agent 7 dissolved in 100 ml of water) for a time period of 60 min, respectively. Afterwards, the materials were immersed repeatedly in fresh ethanol-solution, which was used as an extraction medium for extracting the agent 7 from the materials, and the concentration of the agent 7 in the respective ethanol-solution was measured each time. In other words, at different points in time, fresh ethanol-solutions were used for extracting the agent from the respective thermoplastic material. The graph displays the measured concentration of the agent 7 in the respective solution for each material for an extraction time of 0.7 h, 7 h, and 70 h, respectively.

As the graph shows (note the logarithmic scale on both axes!), the proposed material system (upper two symbols), comprising a cellulose-based cap layer (materials NA750 and NA550, respectively) applied onto a core of a thermoplastic material, can initially load more of the agent 7 from the same reload-liquid 23 and additionally can store the agent 7 for a much longer period of time (thus offering a lower release rate of the agent 7 over time), as compared to a poly-urethane (PU) based thermofoil (Zendura) or a pure PETG-based thermofoil (both without any coating). In bare numbers, the approach according to the invention allows for an increase in release rate by a factor of 2..6 after a reload time of only 60 minutes; the period of time during which a significant release of the agent is maintained (and thus the desired anti-microbial functionality of the dental appliance) is extended by about six times. The uptake of the cellulose-based cap layers is at least a factor of 2..4 higher, as compared to the pure core materials PETG and PU.

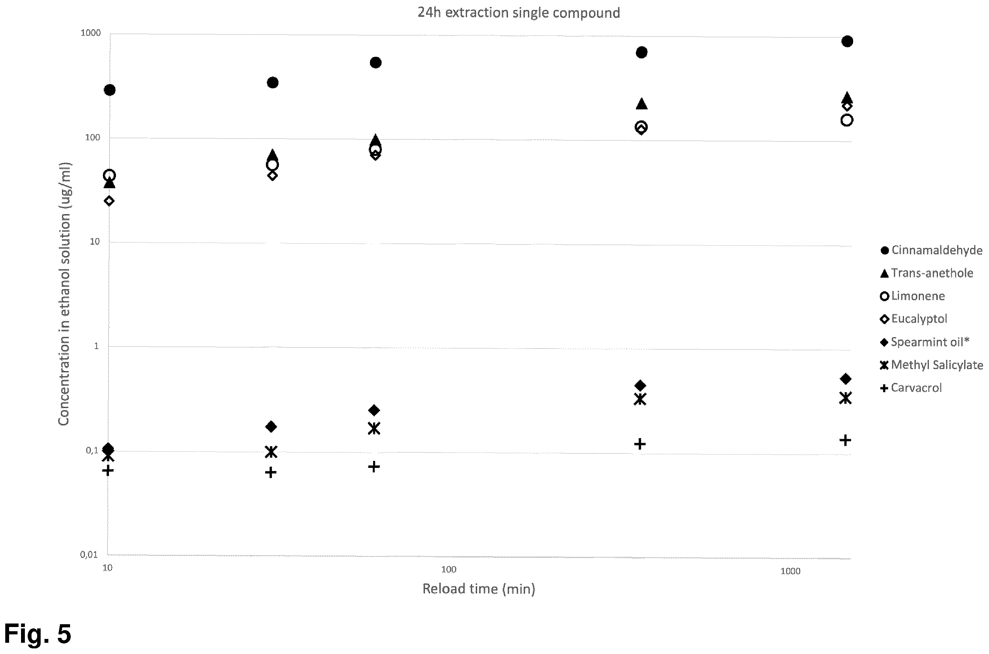

Figure 5 displays experimental data obtained in a second experiment in which dental appliances according to the invention, each time comprising a cellulose-based cap layer, was loaded with 7 different types of agents 7 for time periods ranging from 10 to 1440 min (= 24h). The graph shows the concentration of each agent 7 in 40% ethanol-water-solution, which was used as an extraction medium over a period of 24 hours. Depending on the chemical side group (in particular its polarity) of the particular agent, the capacity to interact with the dental appliance (through storage and release) can vary by a factor of up to 10000, as can be seen by comparing the data for Carvacrol and Cinnamaldehyde. As a result, Cinnamaldehyde appears as an agent that is particularly suited for the applications described herein.

Finally, additional calorimetric experiments were performed to measure the capability of different agents of suppressing the growth of bacteria such as streptococcus mutans and streptococcus mitis. The different agents 7 were applied as a solution containing a single essential oil component at a concentration of 0.1% in BHI. As figure of merits, the lag phase, defined as the postponement in time of the growth of the bacteria and measured in hours, and the growth rate (increase in number of bacteria / time), measured in J/h, of the streptococcus populations were determined as follows:

| agent | growth rate (J/h) of **S. mutans** | lag phase (h) of **S. mutans** growth |
|---|---|---|
| Limonene | 0 | >24 |
| cinnamaldehyde | 0 | >24 |
| trans-anethole | 0.03 | >24 |
| methyl salicylate | 1.2 | 5 |
| Eucalyptol | 1.3 | 5 |

| agent | growth rate (J/h) of **S. mitis** | lag phase (h) of **S. mitis** growth |
|---|---|---|
| methyl salicylate | 0.4 | 23 |
| trans-anethole | 0.13 | 6.4 |
| cinnamaldehyde | 0.15 | 6.2 |
| Limonene | 0.11 | 3 |
| Eucalyptol | 0.14 | 2 |

As can be seen from these data, in particular Limonene and cinnamaldehyde produce lag phases exceeding 24 hours without any measurable growth w.r.t. streptococcus mutans. With respect to streptococcus mitis, methyl salicylate offers the best protection due to a lag phase of 23 hours at a moderate growth rate of 0.4 J/h.

In addition, the difference between non-polar and polar agents should be noted here since the polarity of the agent 7 will define the reload speed into the cellulose-based (e.g. CAB) coating of the dental appliance 2 as well as the extraction speed in the saliva of the patient. Furthermore, the polarities of the agents 5 impact their respective interaction when combined together in one reload-liquid 23 and thus influence the reload speed of each single agent **7,** depending on the ratio to each other and the ratio to the surfactant (if present in the reload-liquid 23).

As cinnamaldehyde is polar, while limonene and methyl salicylate are non-polar, cinnamaldehyde is particularly suited as an anti-microbial agent 7 to be used with a material system as proposed herein with a cellulose-based cap layer, because cinnamaldehyde offers similar anti-microbial protection but superior reload speed.

For achieving best protection against both streptococcus mutans and streptococcus mitis, a combination of Limonene or cinnamaldehyde and methyl salicylate or trans-anethole is proposed to be used as the agent 7 in the reload-liquid 23 described previously and thus as the antimicrobial agent 7 to be delivered by the dental appliance 2 to the user.

### List of reference numerals

- 1: thermoplastic functional foil
- 2: dental appliance
- 3: core
- 4: thermoplastic material
- 5: thermoplastic coating
- 6: organic polymer
- 7: agent
- 8: agent liquid
- 9: carrier liquid
- 10: bio-based material
- 11: carrier solution
- 12: solvent
- 13: core foil
- 14: heat applied
- 15: pre-form
- 16: cavities (for taking up single teeth)
- 17: interface
- 18: outer surface
- 19: non-fossil carbon content (containing ¹⁴C)
- 20: carbon content based on fossil sources (containing no detectable fraction of ¹⁴C any more)
- 21: bio based content (i.e. non-fossil carbon content plus hydrogen, nitrogen, and oxygen bound to this content)
- 22: hydrogen, nitrogen, and oxygen bound to non-fossil carbon
- 23: reload-liquid
- 24: liquid precursor
- 25: tablet
- 26: pipette

## Claims

1. **Method for initially loading or re-loading a dental appliance** (2), such as an orthodontic appliance (2) or a protective appliance (2) for protection of teeth and/or gingiva, with an agent (7) which is releasable from a functional coating (5) of the appliance (2) in aqueous environment,
- wherein the dental appliance (2) features a solid core (3) of thermoplastic material (4), **characterized in that**
- the functional coating (5) of the dental appliance (2) is a thermoplastic coating (5) at least partially covering the core (3) and this functional coating (5) has been thermoformed together with the core (3) to form the dental appliance (2), and
- the agent (7) is initially loaded or re-loaded into the coating (5) by using a reload-liquid (23) that contains the agent (7) and by immersing the dental appliance (2) in the reload-liquid (23) to load or re-load the agent (7) into the coating (5).

2. **Use of a reload-liquid (23)** for re-loading a dental appliance (2) with a releasable agent (7), wherein the reload-liquid (23) contains the agent (7),
**characterized in that**
- the dental appliance (2) is immersed in the reload-liquid (23) to load the agent (7) into a solid thermoplastic coating (5) of the dental appliance (2).

3. Use of a reload-liquid (23) according to the previous claim, wherein the reload-liquid (23) is
- an aqueous solution, in particular containing a solvent such as ethanol;
or
- an oil-based liquid.

4. Use of a reload-liquid (23) according to claim 2 or claim 3, wherein the reload-liquid (23) is obtained by dissolving a solid tablet (25) containing the agent (7) in a liquid,
- preferably wherein the tablet (25) is soluble in water and the liquid is water.

5. Use of a reload-liquid (23) according to any of the claims 2 to 4, wherein the reload-liquid (23) and/or the tablet (25) comprises a surfactant for homogenously distributing the agent (7) within the reload-liquid (23),
- preferably wherein the surfactant is derived from a renewable biological resource, in particular a material of non-fossil origin produced by a living organism,
- most preferably wherein the surfactant comprises one of the following substances or a combination thereof: a fatty acid ester, a benzoic alcohol, a glycerol-based surfactant, Polyglyceryl-4 Laurate, or Polyglyceryl-6 Caprate.

6. Use of a reload-liquid (23) according to claim 5,
- wherein a concentration of the surfactant cₛ in the reload-liquid (23) is cₛ > 1.1 g/l, and/or
- wherein a ratio of a concentration cₛ of the surfactant and a concentration cₐ of the agent (7) in the reload-liquid (23) is cₛ/cₐ < 100, most preferably cₛ/cₐ < 10.

7. Use of a reload-liquid (23) according to any of the claims 2 to 6, wherein the agent (7) is
- an antimicrobial agent (7), preferably cinnamaldehyde, and/or
- derived from a renewable biological resource, in particular a material of non-fossil origin produced by a living organism, and/or
- an essential oil.

8. Use of a reload-liquid (23) according to any of the claims 2 to 7, wherein the agent (7) comprises a combination of the following essential oils:
Limonene or cinnamaldehyde
and
methyl salicylate or trans-anethole.

## Patentansprüche

1. **Verfahren zum anfänglichen Laden oder Nachladen einer Dentalvorrichtung** (2), zum Beispiel einer kieferorthopädischen Vorrichtung (2) oder einer Schutzvorrichtung (2) zum Schutz von Zähnen und/oder Zahnfleisch, mit einem Mittel (7), das aus einer funktionalen Beschichtung (5) der Vorrichtung (2) in wässriger Umgebung freisetzbar ist,
- wobei die Dentalvorrichtung (2) einen soliden Kern (3) aus thermoplastischem Material (4) aufweist, **dadurch gekennzeichnet, dass**
- die funktionale Beschichtung (5) der Dentalvorrichtung (2) eine thermoplastische Beschichtung (5) ist, die zumindest teilweise den Kern (3) abdeckt, und diese funktionale Beschichtung (5) zusammen mit dem Kern (3) dazu thermogeformt wurde, die Dentalvorrichtung (2) zu bilden, und
- das Mittel (7) anfänglich oder in die Beschichtung (5) nachgeladen wird durch Verwenden einer Nachlade-Flüssigkeit (23), welche das Mittel (7) enthält, und durch Eintauchen der Dentalvorrichtung (2) in die Nachlade-Flüssigkeit (23) zum Laden oder Nachladen des Mittels (7) in die Beschichtung (5).

2. **Verwendung einer Nachlade-Flüssigkeit (23)** zum Nachladen einer Dentalvorrichtung (2) mit einem freisetzbaren Mittel (7), wobei die Nachlade-Flüssigkeit (23) das Mittel (7) enthält,
**dadurch gekennzeichnet, dass**
- die Dentalvorrichtung (2) zum Laden des Mittels (7) in eine solide thermoplastische Beschichtung (5) der Dentalvorrichtung (2) in die Nachlade-Flüssigkeit (23) eingetaucht wird.

3. Verwendung einer Nachlade-Flüssigkeit (23) nach dem vorhergehenden Anspruch, wobei die Nachlade-Flüssigkeit (23)
- eine wässrige Lösung ist, die insbesondere ein Lösungsmittel wie zum Beispiel Ethanol enthält;
oder
- eine Flüssigkeit auf Ölbasis ist.

4. Verwendung einer Nachlade-Flüssigkeit (23) nach Anspruch 2 oder Anspruch 3, wobei die Nachlade-Flüssigkeit (23) gewonnen wird durch Lösen einer soliden Tablette (25), die das Mittel (7) enthält, in einer Flüssigkeit,
- wobei die Tablette (25) bevorzugt wasserlöslich ist und die Flüssigkeit Wasser ist.

5. Verwendung einer Nachlade-Flüssigkeit (23) nach einem der Ansprüche 2 bis 4, wobei die Nachlade-Flüssigkeit (23) und/oder die Tablette (25) ein Tensid zum homogenen Verteilen des Mittels (7) in der Nachlade-Flüssigkeit (23) umfasst,
- wobei das Tensid bevorzugt von einer erneuerbaren biologischen Ressource abgeleitet ist, insbesondere einem Material nicht-fossilen Ursprungs, das von einem lebenden Organismus produziert wird,
- wobei das Tensid am bevorzugtesten eine der folgenden Substanzen oder eine Kombination davon umfasst: einen Fettsäureester, einen Benzoesäurealkohol, ein Tensid auf Glycerinbasis, Polyglyceryl-4-Laurat oder Polyglyceryl-6-Caprat.

6. Verwendung einer Nachlade-Flüssigkeit (23) nach Anspruch 5,
- wobei eine Konzentration des Tensids cₛ in der Nachlade-Flüssigkeit (23) cₛ > 1,1 g/l ist und/oder
- wobei ein Verhältnis einer Konzentration cₛ des Tensids und einer Konzentration cₐ des Mittels (7) in der Nachlade-Flüssigkeit (23) cₛ/cₐ < 100, am bevorzugtesten cₛ/cₐ < 10 ist.

7. Verwendung einer Nachlade-Flüssigkeit (23) nach einem der Ansprüche 2 bis 6, wobei das Mittel (7)
- ein antimikrobielles Mittel (7), bevorzugt Zimtaldehyd, ist und/oder
- von einer erneuerbaren biologischen Ressource abgeleitet ist, insbesondere einem Material nicht-fossilen Ursprungs, das von einem lebenden Organismus produziert wird, und/oder
- ein ätherisches Öl ist.

8. Verwendung einer Nachlade-Flüssigkeit (23) nach einem der Ansprüche 2 bis 7, wobei das Mittel (7) eine Kombination aus den folgenden ätherischen Ölen umfasst:
Limonen oder Zimtaldehyd
und
Methylsalicylat oder Trans-Anethol.

## Revendications

1. **Procédé pour charger initialement ou recharger un appareil dentaire** (2), tel qu'un appareil orthodontique (2) ou un appareil de protection (2) pour la protection des dents et/ou des gencives, avec un agent (7) qui est libérable d'un revêtement (5) fonctionnel de l'appareil (2) dans un environnement aqueux,
- dans lequel l'appareil dentaire (2) présente la particularité d'avoir un noyau (3) solide en matériau thermoplastique (4), **caractérisé en ce que**
- le revêtement (5) fonctionnel de l'appareil dentaire (2) est un revêtement thermoplastique (5) recouvrant au moins partiellement le noyau (3) et ce revêtement (5) fonctionnel a été thermoformé conjointement avec le noyau (3) pour former l'appareil dentaire (2), et
- l'agent (7) est initialement chargé ou rechargé dans le revêtement (5) en utilisant un liquide de rechargement (23) qui contient l'agent (7) et en immergeant l'appareil dentaire (2) dans le liquide de rechargement (23) pour charger ou recharger l'agent (7) dans le revêtement (5).

2. **Utilisation d'un liquide de rechargement (23)** pour recharger un appareil dentaire (2) avec un agent (7) libérable, dans laquelle le liquide de rechargement (23) contient l'agent (7),
**caractérisée en ce que**
- l'appareil dentaire (2) est immergé dans le liquide de rechargement (23) pour charger l'agent (7) dans un revêtement (5) thermoplastique solide de l'appareil dentaire (2).

3. Utilisation d'un liquide de rechargement (23) selon la revendication précédente, dans laquelle le liquide de rechargement (23) est
- une solution aqueuse, contenant en particulier un solvant tel que de l'éthanol ; ou
- un liquide à base d'huile.

4. Utilisation d'un liquide de rechargement (23) selon la revendication 2 ou la revendication 3, dans laquelle le liquide de rechargement (23) est obtenu en dissolvant un comprimé (25) solide contenant l'agent (7) dans un liquide,
- de préférence dans laquelle le comprimé (25) est soluble dans l'eau et le liquide est de l'eau.

5. Utilisation d'un liquide de rechargement (23) selon l'une quelconque des revendications 2 à 4, dans laquelle le liquide de rechargement (23) et/ou le comprimé (25) comprend un tensioactif pour répartir de manière homogène l'agent (7) au sein du liquide de rechargement (23),
- de préférence dans laquelle le tensioactif est dérivé d'une ressource biologique renouvelable, en particulier d'un matériau d'origine non fossile produit par un organisme vivant,
- le plus préférentiellement dans laquelle le tensioactif comprend l'une des substances suivantes ou l'une de leurs combinaisons : un ester d'acide gras, un alcool benzoïque, un tensioactif à base de glycérol, le laurate de polyglycéryle-4 ou le caprate de polyglycéryle-6.

6. Utilisation d'un liquide de rechargement (23) selon la revendication 5,
- dans laquelle une concentration du tensioactif cₛ dans le liquide de rechargement (23) est cₛ > 1,1 g/L, et/ou
- dans laquelle un rapport entre une concentration cₛ du tensioactif et une concentration Cₐ de l'agent (7) dans le liquide de rechargement (23) est cₛ/cₐ < 100, le plus préférentiellement cₛ/cₐ < 10.

7. Utilisation d'un liquide de rechargement (23) selon l'une quelconque des revendications 2 à 6, dans laquelle l'agent (7) est
- un agent antimicrobien (7), de préférence le cinnamaldéhyde, et/ou
- dérivé d'une ressource biologique renouvelable, en particulier d'un matériau d'origine non fossile produit par un organisme vivant, et/ou
- une huile essentielle.

8. Utilisation d'un liquide de rechargement (23) selon l'une quelconque des revendications 2 à 7, dans laquelle l'agent (7) comprend une combinaison des huiles essentielles suivantes :
du limonène ou du cinnamaldéhyde
et
du salicylate de méthyle ou du trans-anéthole.
